# EUROPÄISCHE PATENTSCHRIFT

(11) **EP 1 447 110 B1**
(45) Veröffentlichungstag und Bekanntmachung des Hinweises auf die Patenterteilung: **21.11.2007**
(21) Anmeldenummer: 04000685.0
(22) Anmeldetag: 15.01.2004
(51) Int. Cl.: A61M 15/00, B05B 17/04, B05B 17/06

(54) **Mikrodosiereinrichtung**
Micro dosing device
Dispositif de dosage micro

(30) Priorität: 13.02.2003 DE 10306683
(43) Veröffentlichungstag der Anmeldung: 18.08.2004
(73) Patentinhaber: Ing. Erich Pfeiffer GmbH, 78315 Radolfzell (DE)
(72) Erfinder: Körner, Joachim, 88690 Uhldingen (DE); Helminger, Michael, 78315 Radolfzell (DE); Schürle, Holger, 78315 Radolfzell (DE); Bommer, Rene Dr., 78476 Allensbach (DE)
(74) Vertreter: Patentanwälte Ruff, Wilhelm, Beier, Dauster & Partner

(56) Entgegenhaltungen:
- EP-A1- 0 923 957
- US-A- 5 970 974
- US-A- 6 062 212

## Beschreibung

Die Erfindung betrifft eine Mikrodosiereinrichtung mit einem Dosierraum zur zumindest zeitweisen Aufnahme einer Flüssigkeitsmenge, dem wenigstens eine Austragöffnung zugeordnet ist, sowie mit einer Vibrationseinheit, die mit wenigstens einer Begrenzungsfläche des Dosierraumes in Wirkverbindung steht, um diese für einen Austragvorgang in Schwingungen zu versetzen, und mit einer mit der Vibrationseinheit verbundenen Abgabefunktionseinheit zur Aktivierung der Vibrationseinheit während einer Abgabezeitdauer.

Eine derartige Mikrodosiereinrichtung ist aus der EP 0923957 und der US 6 196 219 B1 bekannt. Die bekannte Mikrodosiereinrichtung weist eine Zerstäubereinheit auf, in der ein Dosierraum für die Aufnahme einer abzugebenden Flüssigkeitsmenge vorgesehen ist. Der Dosierraum ist auf einer Seite durch eine mit mehreren Austragöffnungen in Form von Perforationen versehenen Membran verschlossen. Der gegenüberliegenden Begrenzungsfläche ist ein als Vibrationseinheit dienender, piezoelektrischer Aktuator zugeordnet. In den Dosierraum mündet ein Zuführkanal, der die für einen Austragvorgang benötigte Flüssigkeitsmenge aus einem größeren Vorratsspeicher in den Dosierraum führt. Bei einer Aktivierung der Vibrationseinheit, d.h. des piezoelektrischen Aktuators, geraten die Begrenzungsflächen des Dosierraumes und vorzugsweise auch die Membran in Schwingungen, wodurch die im Dosierraum befindliche Flüssigkeitsmenge durch die Austragöffnungen der Membran hindurch zerstäubt und damit ausgebracht wird. Der piezoelektrische Aktuator wird auch nach Beendigung des eigentlichen Austragvorganges weiter betrieben, um den Dosierraum zu erhitzen und so eine Trocknung und Verdampfung etwa noch vorhandener Flüssigkeitsreste vorzunehmen.

Aus der EP 0 682 570 B1 ist eine weitere Dosiereinrichtung bekannt, bei der Flüssigkeit aus einem Vorratsbereich durch eine perforierte Membran hindurch als zerstäubter Sprühnebel nach außen abgegeben wird, indem die Membran durch Vibrationsmittel in Schwingungen versetzt wird. Auch bei dieser Dosiereinrichtung ist es vorgesehen, die Vibrationsmittel länger zu betreiben, als dies durch eine zur Abgabe der dosierten Flüssigkeitsmenge notwendige Zeitdauer erforderlich ist. Dadurch soll die gesamte Flüssigkeitsmenge aus dem Vorratsraum abgegeben werden.

Aufgabe der Erfindung ist es, eine Mikrodosiereinrichtung der eingangs genannten Art zu schaffen, die eine zeitlich exakt definierte Dosierung einer Flüssigkeitsmenge ermöglicht.

Diese Aufgabe wird durch die Vorrichtung gemäß Anspruch 1 gelöst. Durch die zeitliche Trennung der Abgabefunktion und der Trocknungsfunktion ist eine zeitlich klar definierte Dosierbarkeit der entsprechenden Flüssigkeit gewährleistet. Es ist dadurch möglich, das Dosiervolumen über das Zeitintervall der Abgabefunktion vorzugsweise auf gleichem Niveau zu halten, wodurch eine hochgenaue Dosierung ermöglicht ist. Ein Nachlaufen der Vibrationseinheit, wie es im Stand der Technik vorgesehen ist, wird vermieden. Die erfindungsgemäße Lösung eignet sich insbesondere für pharmazeutische Anwendungszwecke, die gegebenenfalls hochgenaue Applikationen erforderlich machen. Die erfindungsgemäße Lösung ist insbesondere auch einsetzbar für kosmetische Zwecke oder für die Beduftung von mobilen und immobilen Räumen. Die Trocknungsfunktion wird erfindungsgemäß zeitlich versetzt zu dem Applikationszeitraum vorgenommen, so dass etwaige, während des Trocknungsvorganges nach außen gelangende Flüssigkeitsreste nicht mehr in den menschlichen Körper gelangen, sondern vorzugsweise an die Umgebung abgegeben werden. Vorzugsweise arbeitet die Vibrationseinheit mit Ultraschallwellen, indem ein piezoelektrischer Aktuator mit entsprechender Frequenz angeregt wird. Die Vibrationseinheit kann auch Aktuatoren aufweisen, die mit anderen Erregerschwingungen oder -wellen arbeiten.

Die erfindungsgemäße Lösung ist besonders vorteilhaft einsetzbar bei hochkonzentrierten, pharmazeutischen Wirkstoffen, bei denen geringste Überdosierungen bereits zu nachteiligen Folgen für den menschlichen Körper führen können, oder bei denen schädliche Kontaminationen der Flüssigkeit auftreten können. Die Trocknungsfunktionseinheit kann entweder - wie auch die Abgabefunktionseinheit - mit der Vibrationseinheit in Verbindung stehen und auf diese einwirken, oder auch als von der Vibrationseinheit vollkommen unabhängige Funktionseinrichtung gestaltet sein. Die erfindungsgemäße Lösung eignet sich insbesondere für eine Mikrodosiereinrichtung mit Zerstäubungsfunktion, bei der die Flüssigkeitsmenge durch winzige Austragöffnungen einer Membran hindurch als zerstäubter Sprühnebel ausgebracht wird. In gleicher Weise ist die erfindungsgemäße Mikrodosiereinrichtung auch für Mikrodosierpumpen anderer Art einsetzbar, bei der kein zerstäubter Flüssigkeitsaustrag erfolgt.

In Ausgestaltung der Erfindung steht die Trocknungsfunktionseinheit mit der Vibrationseinheit in Verbindung, um diese für einen Trocknungsvorgang zu aktivieren. Diese Ausgestaltung ist insbesondere vorteilhaft, falls als Vibrationseinheit ein piezoelektrischer Aktuator vorgesehen ist. Denn bei geeigneter Schwingungsfrequenz auf die wenigstens eine Begrenzungsfläche des Dosierraumes ist eine Aufheizung des Dosierraumes erzielbar, die zu einer Verdampfung noch vorhandener Flüssigkeitsreste führt.

In weiterer Ausgestaltung der Erfindung sind die Abgabefunktionseinheit und die Trocknungsfunktionseinheit Teile einer gemeinsamen elektronischen Steuereinrichtung, die vorzugsweise mit einem Zeitfunktionsglied zur Koordination der zeitlich getrennten Aktivierungsvorgänge der Vibrationseinheit durch die Abgabefunktionseinheit und die Trocknungsfunktionseinheit versehen ist. Die Integration der Abgabefunktionseinheit und der Trocknungsfunktionseinheit in einer elektronischen Steuereinrichtung ermöglicht eine äußerst kleine Bauweise. Dennoch sind hochpräzise Schaltungs- oder Aktivierungsvorgänge insbesondere des piezoelektrischen Aktuators erzielbar. Das Zeitfunktionsglied gewährleistet die zeitliche Trennung zwischen der Abgabefunktion und der Trocknungsfunktion. Vorzugsweise ist die Trocknungsfunktion der Abgabefunktion zeitlich nachgeschaltet.

In weiterer Ausgestaltung der Erfindung ist dem Dosierraum ein Auffangspeicher zum Aufnehmen der Flüssigkeitsreste aus dem Dosierraum in gasförmigem oder flüssigem Zustand zugeordnet. Diese Ausgestaltung verhindert das unkontrollierte Abgeben der Flüssigkeitsreste an die Umgebungsluft. Das Auffangen der Flüssigkeitsreste in dem Auffangspeicher ermöglicht eine äußerst saubere Entsorgung dieser Flüssigkeitsreste. Darüber hinaus kann gegebenenfalls eine Wiederverwertung der aufgefangenen Flüssigkeitsreste vorgenommen werden.

In weiterer Ausgestaltung der Erfindung umfasst die Trocknungsfunktionseinheit eine Heizeinrichtung oder eine Fördereinrichtung zum Herauspumpen oder Heraussaugen der Flüssigkeitsreste. Diese Ausgestaltungen bilden für die Trocknungsfunktionseinheit von der Abgabefunktionseinheit unabhängige Funktionseinrichtungen, die je nach Einsatzzweck wahlweise zur Anwendung kommen können.

Die Erfindung betrifft zudem ein Verfahren zum Dosieren kleiner Flüssigkeitsmengen durch Vibration von wenigstens einer Begrenzungsfläche eines Dosierraumes mittels einer Aktivierung und Deaktivierung einer Vibrationseinheit.

Aufgabe der Erfindung ist es somit auch, ein derartiges Verfahren zu schaffen, das eine besonders exakte Dosierung insbesondere bei Mehrfachanwendungen ermöglicht.

Diese Aufgabe wird durch das Verfahren gemäß Anspruch 6 gelöst. Das erfindungsgemäße Verfahren eignet sich insbesondere für die Abgabe der Flüssigkeit als zerstäubter Sprühnebel. Durch die erfindungsgemäße Lösung ist eine besonders exakte und zeitlich definierte Dosierung erzielbar. Durch die unabhängige und vorzugsweise vollständige Trocknung des Dosierraumes vor einem erneuten Flüssigkeitsaustrag sind auch bei Mehrfachapplikationen jeweils exakte Dosierungen möglich. Kontaminationen einer erneuten Dosiermenge durch Flüssigkeitsreste der zuvor ausgebrachten Dosiermenge werden vermieden. Wesentlich ist es erfindungsgemäß, in den für das Risiko einer Kontamination maßgeblichen Bereichen des Dosierraumes eine gute Trocknung zu erzielen. Eine vollständige Trocknung in von diesen maßgeblichen Bereichen abliegenden Abschnitten ist nicht unbedingt notwendig.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt schematisch in einer Schnittdarstellung eine Ausführungsform einer erfindungsgemäßen Mikrodosiereinrichtung in Form einer Zerstäubereinheit,
- Fig. 2: ein Blockdiagramm für die Funktion der Zerstäubereinheit nach Fig. 1 und
- Fig. 3: eine weitere Mikrodosiereinrichtung ähnlich Fig. 1, ebenfalls in schematischer Schnittdarstellung.

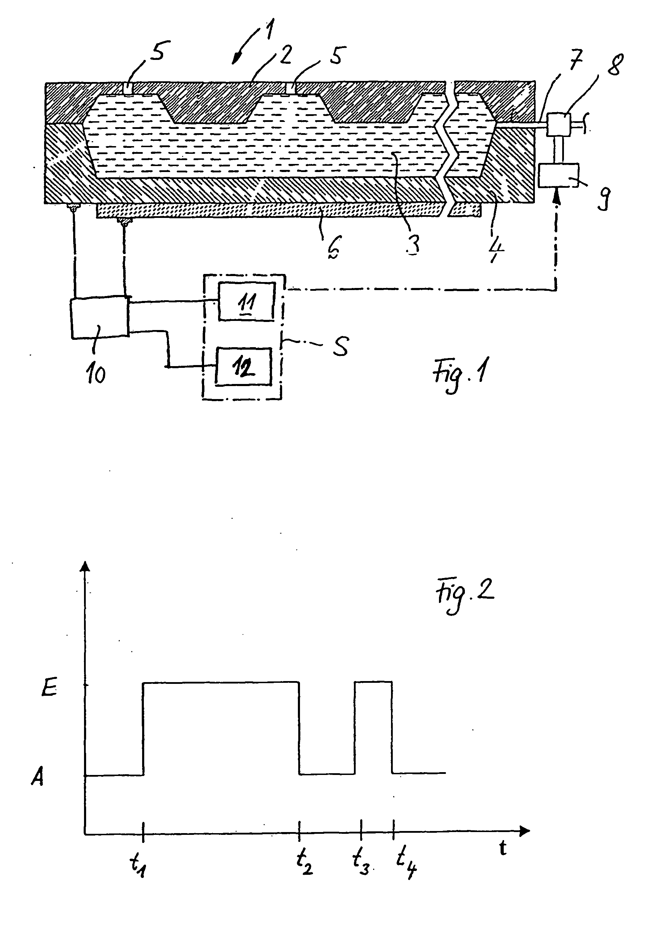

Eine Mikrodosiereinrichtung nach Fig. 1 ist als Zerstäubereinheit ausgeführt und entspricht vom grundsätzlichen Aufbau her einer Mikrodosiereinheit, wie sie aus der US 6 196 219 B1 bekannt ist. Die Zerstäubereinheit 1 ist Teil eines Inhalators für medizinische Zwecke, um pharmazeutische Wirkstoffe in flüssiger Form über die Atemwege in den menschlichen Körper einzubringen. In gleicher Weise kann die Zerstäubereinheit aber auch für andere Anwendungszwecke und Anwendungsgebiete eingesetzt werden.

Die Zerstäubereinheit 1 weist einen Dosierraum 3 auf, der nach oben durch eine Membran 2 und nach unten durch eine Bodenstruktur 4 begrenzt ist. Die Bodenstruktur 4 ist vorzugsweise aus Glas, Metall, Keramik, Silizium, einem Piezokristall, einem hochverdichteten Polymer oder ähnlichem hergestellt. Die als Deckstruktur dienende Membran 2 für den Dosierraum 3 ist vorzugsweise aus Kunststoff, Keramik, Metall, Silizium oder ähnlichem hergestellt. Die Membran 2 weist nach Art einer flächigen Perforierung mehrere winzige Austragöffnungen 5 auf, die den Dosierraum 3 zu einer Umgebung hin öffnen. Wenigstens im Bereich der düsenartigen Austragöffnungen 5 ist die Membran 2 vorzugsweise aus Silizium hergestellt. Die Membran 2 und die Bodenstruktur 4 sind vorzugsweise umlaufend miteinander verbunden, um einen geschlossenen Dosierraum 3 zu erzielen.

An einer Unterseite der Bodenstruktur 4 ist ein piezoelektrischer Aktuator 6 angeordnet. Im Verbindungsbereich zwischen der Membran 2 und der Bodenstruktur 4 ist ein in den Dosierraum 3 mündender Zuführkanal 7 vorgesehen, dem ein Mikroventil 8 für ein Öffnen und Schließen des Zuführkanals 7 zugeordnet ist. Das Mikroventil 8 ist durch ein Betätigungsglied 9 beaufschlagbar, das wiederum durch eine elektronische Steuereinheit S angesteuert wird.

Der Zuführkanal 7 steht in nicht näher dargestellter Weise mit einer Vorratskammer für die Flüssigkeit in Verbindung. Der Dosierraum 3 weist vorzugsweise ein Fassungsvolumen von 2 µl bis 3 µl auf. Die Zufuhr von Flüssigkeit aus der Vorratskammer in den Dosierraum 3 erfolgt vorzugsweise entweder durch leichten Überdruck im Bereich der Vorratskammer und/oder durch Kapillarwirkungen.

Der Dosierraum 3 weist im Bereich jeder Austragöffnung 5 eine sich in Austragsrichtung konisch verjüngende Kavität auf. Die Austragsöffnungen 5 selbst weisen jeweils eine zylindrische Wandung auf. Die Durchmesser der Austragsöffnungen 5 sind wesentlich geringer als die Durchmesser der konischen Kavitäten am Übergang jedes kegelstumpfförmigen Abschnittes zu der jeweils zylindrischen Austragöffnung 5.
Um einen Austragvorgang zu ermöglichen, ist die untere Begrenzungsfläche des Dosierraumes 3, vorliegend die Bodenstruktur 4, durch einen piezoelektrischen Aktuator 6 in Schwingungen versetzbar, die vorzugsweise im hochfrequenten Bereich liegen. Der piezoelektrische Aktuator 6 ist plattenförmig gestaltet und mittels einer Elektrode mit einem elektrischen oder elektronischen Steuerelement 10 verbunden. Das elektrische oder elektronische Steuerelement 10 ist mittels einer weiteren Elektrode an eine Bodenseite der Bodenstruktur 4 angeschlossen. Das Steuerelement 10 steht zum einen mit einer Abgabefunktionseinheit 12 und zum anderen mit einer Trocknungsfunktionseinheit 11 in Verbindung. Sowohl die Trocknungsfunktionseinheit 11 als auch die Abgabefunktionseinheit 12 sind vorliegend Teil einer schematisch dargestellten, elektronischen Steuereinheit S, die mittels eines nicht näher dargestellten Zeitfunktionsgliedes alternativ eine Aktivierung des Steuerelementes 10 durch die Abgabefunktionseinheit 12 oder durch die Trocknungsfunktionseinheit 11 ermöglicht. Vorzugsweise sind sowohl die Trocknungsfunktionseinheit 11 als auch die Abgabefunktionseinheit 12 jeweils als geeignete elektronische Bauteile gestaltet. Alternativ ist es auch möglich, die Trocknungsfunktionseinheit 11 und die Abgabefunktionseinheit 12 im Rahmen einer Softwarestruktur innerhalb einer elektronischen Baueinheit als getrennte Funktionseinheiten zu kreieren. Sowohl die Trocknungsfunktionseinheit 11 als auch die Abgabefunktionseinheit 12 sind derart gestaltet, dass sie alternativ das Steuerelement 10 und damit den piezoelektrischen Aktuator 6 jeweils für eine bestimmte Zeitdauer einschalten und anschließend wieder ausschalten. Anhand des Blockdiagramms in Fig. 2 ist das zeitabhängige Zu- und Abschalten der Abgabefunktionseinheit 12 und der Trocknungsfunktionseinheit 11 erläutert. Mit dem Bezugszeichen A ist der ausgeschaltete, d.h. deaktivierte Zustand des piezoelektrischen Aktuators 6 und mit dem Bezugszeichen E der eingeschaltete und damit aktivierte Funktionszustand des piezoelektrischen Aktuators 6 bezeichnet. Auf der Abszisse des ebenen Koordinatensystems ist die Zeit t dargestellt. Die Ordinate stellt - wie bereits beschrieben - den Aktivierungs- oder Funktionszustand des piezoelektrischen Aktuators dar. Im Zeitraum von t₁ bis t₂ wird der Aktuator 6 durch die Abgabefunktionseinheit 12 aktiviert. In diesem Zeitraum wird ein für eine entsprechende Applikation notwendiges Dosiervolumen zerstäubt und den entsprechenden Atemwegen des Menschen zugeführt. Das Dosiervolumen beträgt vorzugsweise zwischen 10 µl und 30 µl. Während des Zerstäubungsvorganges im Zeitraum t₁ bis t₂ ist das Mikroventil 8 über das Betätigungsglied 9 geöffnet. Ab dem Zeitpunkt t₂ bis zu einem Zeitpunkt t₃ wird der piezoelektrische Aktuator 6 ausgeschaltet, d.h. deaktiviert. Um möglicherweise innerhalb des Dosierraumes 3 noch vorhandene, kleine Flüssigkeitsreste zu entfernen, bevor erneut über den Zuführkanal 7 und das Mikroventil 8 eine entsprechende Flüssigkeitsmenge zugeführt wird, wird ab dem Zeitpunkt t₃ der piezoelektrische Aktuator 6 durch die Trocknungsfunktionseinheit 11 aktiviert, d.h. eingeschaltet. Da die Bodenstruktur 4 und die Membran 2 durch die Aktivierung des piezoelektrischen Aktuators 6 im wesentlichen trocken schwingen, erhitzt sich der Dosierraum 3, wodurch die noch vorhandenen Flüssigkeitsreste verdampfen und durch die Austragöffnungen 5 ausgebracht werden. Die Trocknungsfunktionseinheit 11 aktiviert den piezoelektrischen Aktuator 6 bis zum Zeitpunkt t₄ und schaltet ihn dann aus, d.h. deaktiviert ihn. Nun kann erneut Flüssigkeitsmenge aus der Vorratskammer in den Dosierraum 3 zugeführt werden. Eine Kontamination der aus der geschlossenen Vorratskammer zugeführten Flüssigkeit durch verbliebene Flüssigkeitsreste des vorherigen Dosiervorganges wird vermieden, da diese Flüssigkeitsreste durch den vorangegangenen Trocknungsvorgang entfernt worden sind. Nun kann ein erneuter Dosiervorgang erfolgen, der dann erneut einen entsprechenden Trocknungsvorgang zeitlich getrennt nach sich zieht.

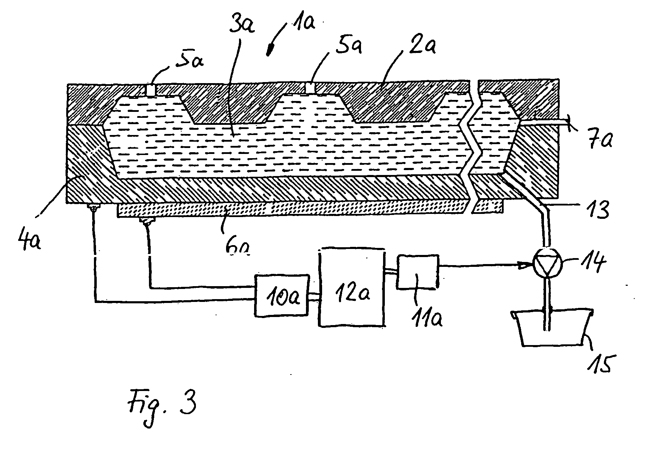

Die Zerstäubereinheit 1a gemäß Fig. 3 entspricht im wesentlichen der zuvor anhand der Fig. 1 und 2 beschriebenen Zerstäubereinheit 1, so dass nachfolgend lediglich noch auf die Unterschiede eingegangen wird. Wesentlicher Unterschied bei der Zerstäubereinheit 1a nach Fig. 3 ist es, dass die Entfernung etwa vergebener Flüssigkeitsreste nach einem Dosiervorgang nicht durch eine erneute Aktivierung des piezoelektrischen Aktuators 6a, sondern vielmehr durch Aktivierung einer separaten Trocknungsfunktionseinheit 11a, die eine Saugeinrichtung 14 umfasst, erfolgt. Funktions- oder baugleiche Teile weisen die gleichen Bezugszeichen auf wie bei der Zerstäubereinheit nach Fig. 1, wobei lediglich zur besseren Unterscheidbarkeit das Bezugszeichen a ergänzt wurde. In der Bodenstruktur 4a ist zusätzlich zu den mit der Zerstäubereinheit 1 baugleichen Elementen oder Abschnitten ein Absaugkanal 13 vorgesehen, der im Bodenbereich des Dosierraumes 3a beginnt, durch die Bodenstruktur 4a aus dieser herausführt und in einen Auffangspeicher 15 mündet. Dem Absaugkanal 13 ist eine Saugpumpe 14 zugeordnet, die vorzugsweise als Mikropumpe gestaltet ist. Die Mikropumpe 14 wird durch die Trocknungsfunktionseinheit 11a aktiviert, die vorzugsweise analog der Darstellung nach Fig. 1 als elektronische Funktions- oder Steuerkomponente gestaltet ist und über ein Zeitfunktionsglied (nicht näher dargestellt) zeitabhängig versetzt zu der Aktivierung und Deaktivierung der Abgabefunktionseinheit 12a fungiert. Ein Aktivierungs- und Deaktivierungsschaubild der Funktion der Zerstäubereinheit 1a entspricht dem Schaubild nach Fig. 2. Nach Beendigung des Dosiervorganges aktiviert die Trocknungsfunktionseinheit 11a die Mikropumpe 14, die in dem Dosierraum 3a einen Unterdruck erzeugt und so etwa noch vorhandene Flüssigkeitsreste durch den Absaugkanal 13 absaugt und in den geschlossenen Auffangspeicher 15 abführt.

## Patentansprüche

1. Mikrodosiereinrichtung mit einem Dosierraum zur zumindest zeitweisen Aufnahme einer Flüssigkeitsmenge, dem wenigstens eine Austragöffnung zugeordnet ist, sowie mit einer Vibrationseinheit, die mit wenigstens einer Begrenzungsfläche des Dosierraumes in Wirkverbindung steht, um diese für einen Austragvorgang in Schwingungen zu versetzen, und mit einer mit der Vibrationseinheit verbundenen Abgabefunktionseinheit zur Aktivierung der Vibrationseinheit während einer Abgabezeitdauer,
**dadurch gekennzeichnet, dass**
zusätzlich eine Trocknungsfunktionseinheit (11, 11a) vorgesehen ist, die zeitlich **durch eine Ruhepause** getrennt **nach Deaktivierung** der Abgabefunktionseinheit (12, 12a) aktiviert wird, um den Dosierraum (3, 3a) von Flüssigkeitsresten zu befreien.

2. Mikrodosiereinrichtung nach Anspruch 1, **dadurch gekennzeichnet, dass** die Trocknungsfunktionseinheit (11) mit der Vibrationseinheit (6) in Verbindung steht, um diese für einen Trocknungsvorgang zu aktivieren.

3. Mikrodosiereinrichtung nach Anspruch 1, **dadurch gekennzeichnet, dass** die Abgabefunktionseinheit (12) und die Trocknungsfunktionseinheit (11) Teile einer gemeinsamen elektronischen Steuereinrichtung (S) sind, die mit einem Zeitfunktionsglied zur Koordination der zeitlich getrennten Aktivierungsvorgänge der Vibrationseinheit (6) durch die Abgabefunktionseinheit (12) und die Trocknungsfunktionseinheit (11) versehen ist.

4. Mikrodosiereinrichtung nach Anspruch 1, **dadurch gekennzeichnet, dass** dem Dosierraum (3) ein Auffangspeicher (15) zum Aufnehmen der Flüssigkeitsreste aus dem Dosierraum (3) in gasförmigem oder flüssigem Zustand zugeordnet ist.

5. Mikrodosiereinrichtung nach Anspruch 1, **dadurch gekennzeichnet, dass** die Trocknungsfunktionseinheit (11a) eine Heizeinrichtung oder eine Fördereinrichtung (14) zum Herauspumpen oder Heraussaugen der Flüssigkeitsreste umfasst.

6. Verfahren zum Dosieren kleiner Flüssigkeitsmengen durch Vibration von wenigstens einer Begrenzungsfläche eines Dosierraumes mittels einer Aktivierung und Deaktivierung einer Vibrationseinheit, **dadurch gekennzeichnet, dass** die Vibrationseinheit (6, 6a) für eine Abgabezeitdauer zum Ausbringen der vorgegebenen Flüssigkeitsmenge aktiviert und anschließend deaktiviert wird, und dass nach der Deaktivierung der Vibrationseinheit (6, 6a) und Verstreichen einer Ruhepause im Dosierraum (3, 3a) verbliebene Flüssigkeitsreste aus dem Dosierraum (3) durch einen Trocknungsvorgang entfernt werden.

7. Verfahren nach Anspruch 6, **dadurch gekennzeichnet, dass** für den Trocknungsvorgang die Vibrationseinheit (6) erneut über eine Trocknungszeitdauer aktiviert wird.

## Claims

1. Micro-dosing device having a dosing chamber for the at least intermittent reception of a liquid amount, at least one discharge opening being allocated to the dosing chamber, and having a vibrating unit which is operatively connected to at least one delimiting surface of the dosing chamber in order to impart vibrations thereto for a discharge process, and having a dispensing function unit connected to the vibrating unit in order to activate the vibrating unit during a dispensing time period, **characterised in that** a drying function unit (11, 11a) is also provided which is activated after deactivation of the dispensing function unit (12, 12a), and then after a rest period, in order to remove liquid residues from the dosing chamber (3, 3a).

2. Micro-dosing device as claimed in Claim 1, **characterised in that** the drying function unit (11) is connected to the vibrating unit (6) in order to activate same for a drying process.

3. Micro-dosing device as claimed in Claim 1, **characterised in that** the dispensing function unit (12) and the drying function unit (11) are parts of a common electronic control device (S) which is provided with a time function element for co-ordinating the temporally separated activating processes of the vibrating unit (6) by the dispensing function unit (12) and the drying function unit (11).

4. Micro-dosing device as claimed in Claim 1, **characterised in that** a collecting reservoir (15) is allocated to the dosing chamber (3) for receiving the liquid residues from the dosing chamber (3) in a gaseous or liquid state.

5. Micro-dosing device as claimed in Claim 1, **characterised in that** the drying function unit (11a) contains a heating device or aconveying device (14) for pumping-out or sucking-out the liquid residues.

6. Method for dosing small amounts of liquid by vibrating at least one delimiting surface of a dosing chamber by means of activating and deactivating a vibrating unit, **characterised in that** the vibrating unit (6, 6a) is activated for a dispensing time period for yielding the predetermined amount of liquid and is then deactivated, and **in that** after deactivation of the vibrating unit (6, 6a) and after a rest period has elapsed, liquid residues remaining in the dosing chamber (3, 3a) are removed from the dosing chamber (3) by a drying process.

7. Method as claimed in Claim 6, **characterised in that** the vibrating unit (6) is reactivated during a drying time period for the drying process.

## Revendications

1. Dispositif de microdosage avec un compartiment de dosage pour la réception au moins temporaire d'une quantité de liquide auquel est associée au moins une ouverture de déversement et avec un ensemble de vibration qui est en communication active avec au moins une surface de délimitation du compartiment de dosage pour faire vibrer celle-ci en vue d'une opération de déversement et avec un ensemble fonctionnel de distribution relié à l'ensemble de vibration pour l'activation de l'ensemble de vibration pendant une période de distribution, **caractérisé en ce qu'**il est prévu, en plus, un ensemble fonctionnel de séchage (11, 11a) qui est activé périodiquement par un temps de repos séparément après la désactivation de l'ensemble fonctionnel de distribution (12, 12a) pour débarrasser le compartiment de dosage (3, 3a) des résidus de liquide.

2. Dispositif de microdosage selon la revendication 1, **caractérisé en ce que** l'ensemble fonctionnel de séchage (11) est en communication avec l'ensemble de vibration (6) pour activer celui-ci en vue d'une opération de séchage.

3. Dispositif de microdosage selon la revendication 1, **caractérisé en ce que** l'ensemble fonctionnel de distribution (12) et l'ensemble fonctionnel de séchage (11) font partie d'un dispositif de commande électronique commun (S) qui est muni d'un organe fonctionnel de temporisation pour la coordination des opérations d'activation de l'ensemble de vibration (6) séparées dans le temps à l'aide de l'ensemble fonctionnel de distribution (12) et de l'ensemble fonctionnel de séchage (11).

4. Dispositif de microdosage selon la revendication 1, **caractérisé en ce qu'**un réservoir collecteur (15) est associé au compartiment de dosage (3) pour la réception des résidus de liquide sortant du compartiment de dosage (3) à l'état gazeux ou liquide.

5. Dispositif de microdosage selon la revendication 1, **caractérisé en ce que** l'ensemble fonctionnel de séchage (11a) comprend un dispositif de chauffage ou un dispositif de refoulement (14) pour le pompage ou l'aspiration des résidus de liquide.

6. Procédé pour le dosage de petites quantités de liquide par la vibration d'au moins une surface de délimitation d'un compartiment de dosage à l'aide d'une activation et d'une désactivation d'un ensemble de vibration, **caractérisé en ce que** l'ensemble de vibration (6, 6a) est activé puis désactivé pendant une période de distribution pour déverser la quantité de liquide prédéterminée et **en ce que**, après la désactivation de l'ensemble de vibration (6, 6a) et l'écoulement d'un temps de repos, les résidus de liquide restés dans le compartiment de dosage (3, 3a) sont extraits du compartiment de dosage (3) par une opération de séchage.

7. Procédé selon la revendication 6, **caractérisé en ce que**, pour l'opération de séchage, l'ensemble de vibration (6) est de nouveau activé pendant une période de séchage.
